# EUROPÄISCHE PATENTSCHRIFT

(11) **EP 1 961 406 B1**
(45) Veröffentlichungstag und Bekanntmachung des Hinweises auf die Patenterteilung: **16.03.2011**
(21) Anmeldenummer: 08405007.9
(22) Anmeldetag: 10.01.2008
(51) Int. Cl.: A61G 12/00, H01H 13/16

(54) **Schaltvorrichtung in einer Bettmatraze**
Switching device in a bed mattress
Dispositif de commutation dans un matelas pour lit

(30) Priorität: 22.02.2007 CH 2972007
(43) Veröffentlichungstag der Anmeldung: 27.08.2008
(60) Teilanmeldung: 10161885.8
(73) Patentinhaber: Polat, Candemir, 4106 Therwil (CH)
(72) Erfinder: Polat, Candemir, 4106 Therwil (CH)
(74) Vertreter: Braun, André jr.

(56) Entgegenhaltungen:
- EP-A- 0 734 004
- US-A- 2 860 595
- US-A- 3 781 843
- US-A- 3 961 201
- US-A- 4 067 005
- US-A- 4 484 043

## Beschreibung

In Spitälern, Pflegeheimen, Rekonvaleszenz-Stationen und ähnlichen Institutionen sind die Betten der Patienten gewöhnlich mit einer zur Erzeugung eines Signals dienenden elektrischen Schaltvorrichtung ausgerüstet. Bei diesen Vorrichtungen handelt es sich üblicherweise um einen durch die im Bett liegende Person leicht erreichbaren, von Hand betätigbaren Drucktastenschalter, der entweder oberhalb des Bettes aufgehängt sein oder auf dem Nachttisch liegen kann, oder um zwei derartige Schalter, von denen je einer so an einer Seite des Bettes angebracht ist, dass eine im Bett liegende Person bei Bedarf mit einer Hand einen der Schalter betätigen kann. Solche Schalter dienen dann zum Einschalten einer Signal- oder Alarmanlage, die sich nicht im gleichen Zimmer wie das Bett befindet, damit die im Bett liegende Person bei jedem Bedürfnis Hilfe herbeirufen kann, sei es beispielsweise um mehr oder weniger zugedeckt zu werden oder um eine Speise oder ein Getränk oder die Hilfe zum Verlassen des Bettes oder irgend etwas anderes zu erhalten.

Es gibt nun aber nicht selten Fälle, bei denen die im Bett liegende Person nicht in der Lage oder nicht willens ist, den Schalter zu betätigen, obwohl die Notwendigkeit dafür vorhanden ist. Dies ist nicht nur bei Personen der Fall, die eine Veranlagung zum Schlafwandeln aufweisen oder die beim Erwachen oder im allgemeinen bei Dunkelheit Mühe haben, sich zurecht zu finden, sondern insbesondere bei all den Personen, die nicht daran denken können, dass sie sich melden sollten, wenn sie aus irgend einem Grund oder auch grundlos das Bett verlassen wollen. Zu diesen Personen gehören unter anderem auch solche, die an Demenz, insbesondere an Altersdemenz leiden. All diese Personen benötigen eine zur Erzeugung eines Signals bestimmte Schaltvorrichtung, die bereits dadurch aktiviert wird, dass die im Bett befindliche Person das Bett verlässt, also ohne dass sie die Schaltvorrichtung vorsätzlich betätigt. Selbstverständlich kann eine solche Schaltvorrichtung mit einer der vorgenannten vorsätzlich betätigbaren Schaltvorrichtungen parallel geschaltet sein.

Zum vorgenannten Zweck sind nun bereits Fussmatten oder Bettvorlagen bekannt, die einen oder mehrere parallel geschaltete Elektroschalter enthalten, von denen mindestens einer dadurch aktiviert, d.h. geschlossen wird, dass eine das Bett verlassenden Person darauf tritt. Solche Bettvorlagen haben aber nicht nur den Nachteil, dass sie nur dann erwartungsgemäss arbeiten, wenn sie am richtigen Ort liegen, daher auch bei grosser Aufmerksamkeit des Pflegepersonals nicht stets "betriebsbereit" sind, sondern noch weitere, ebenfalls bekannte Nachteile sowohl hygienischer wie praktischer Art: Insbesondere bei dementen Patienten müssen sie sehr oft gereinigt und nicht selten sorgfältigst desinfiziert oder gar sterilisiert werden, was nicht nur arbeitsintensiv und teuer ist sondern auch ein Herstellungsmaterial voraussetzt, bei welchem eine derartige Behandlung überhaupt möglicht ist. Der Nachteil im praktischen Gebrauch besteht darin, dass solche Schaltvorrichtungen natürlich auch dann ansprechen, wenn sie von einer sich dem Bett nähernden Pflegeperson oder einem Besucher betreten werden, wenn sie nicht vorher weggenommen werden, wobei das eine wie das andere natürlich höchst unerwünscht ist.

Das Dokument US 4 067 005 offenbart die Merkmale des Oberbegriffs des Anspruchs 1.

All diese Nachteile weist die Schaltvorrichtung nach der vorliegenden Erfindung nicht auf. Sie ist gekennzeichnet durch die im Anspruch 1 genannten Merkmale. Spezielle Ausführungsformen weisen die in den abhängigen Ansprüchen angegebenen Merkmale auf.

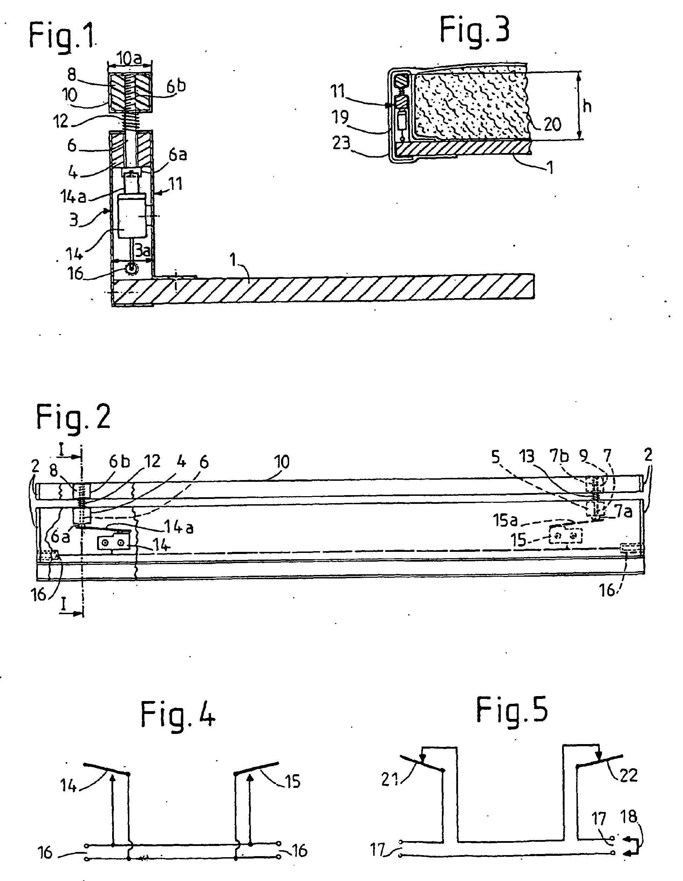

Nachfolgend wird anhand der beiliegenden Zeichnung ein Ausführungsbeispiel der Erfindung beschrieben. In der Zeichnung zeigt
- welche Fig.1: einen Schnitt in grösserem Massstab nach der Linie I -I der in
- der Fig.2: dargestellten Seiten-Ansicht, in welcher aber einzelne Teile der Gehäusewand weggebrochen sind,
- die Fig.3: die Vorrichtung in der Gebrauchsstellung und
- die Fig.4 und 5: das elektrische Schaltschema der als Einschaltvorrichtung bzw. als Ausschaltvorrichtung ausgebildeten Vorrichtung.

Wie insbesondere aus den Figuren 1 und 3 ersichtlich ist, weist das im Querschnitt L-förmige Ausführungsbeispiel als horizontalen Schenkel eine ca.80 cm lange, ca.25 cm breite und ca. 1,5 cm dicke, aus Holz oder einem Kunststoff bestehende Platte 1 auf. An deren einer Längsseite ist als unterer Teil des vertikalen Schenkels 11 ein an seinen beiden Längsenden durch je eine Kunststoffkappe 2 abgeschlossenes Aluminium-Gehäuse 3 befestigt. In dessen obern Bereich sind zwei Führungshülsen 4 und 5 für je einen in ihr verschiebbaren Bolzen 6 bzw. 7 eingeklemmt. Jeder dieser Bolzen weist am untern Ende einen Kopf 6a bzw. 7a und im obern Bereich ein Gewinde 6b bzw.7b auf. Mit diesem ist er in einen beispielsweise aus Kunststoff bestehenden Mutterteil 8 bzw. 9 eingeschraubt, welcher unverdrehbar in einem hohlen Aluminium-Profilstab 10 mit wenigstens angenähert quadratischem Querschnitt sitzt, wobei dessen Breite 10a der Breite 3a des Gehäuses 3 entspricht, sodass er den oberen Teil des vertikalen Schenkels 11 bildet. Die beiden zwischen dem obern und dem untern Teil befindlichen Wendel-Federn 12 und 13, von denen die eine den Bolzen 6 und die andere den Bolzen 7 umgibt, gewährleisten einen horizontalen Zwischenraum zwischen den beiden Teilen und machen es möglich, dass der obere Teil 10 entgegen der Kraft der Federn nach unten verschoben oder verschwenkt werden kann.

Bei der Herstellung des Gehäuses 3 ist seine Höhe so zu bemessen, dass die Höhe h des ganzen Vertikalschenkels 11 mit der Dicke h der Matratze 20 des Bettes, für welches die Schaltvorrichtung bestimmt ist, übereinstimmt. Das sind üblicherweise, jedoch nicht immer, ca.12 cm.

Im Gehäuse 3 sind zwei Elektroschalter 14 und 15 so angeordnet, dass sich ihre Schaltorgane 14a bzw.15a sozusagen abstandsfrei unterhalb der Bolzenköpfe 6a bzw. 7a befinden. Dabei sind die Federn so bemessen, dass bereits ein geringes auf den Profilstab vertikal einwirkendes Gewicht von beispielsweise 5 kg ein Zusammendrücken einer oder beider Federn bewirkt, welches ausreicht, dass wenigstens einer der beiden Elektroschalter 14 oder 15 anspricht.
Falls die Schaltvorrichtung zum Einschalten des Stromes für eine Signal- oder Alarm-Anlage vorgesehen ist, müssen als Schalter 14 und 15 Einschalter verwendet werden, die parallel geschaltet sind, wie das in der Fig.4 dargestellt ist. In eine der beiden mit 16 bezeichneten Steckdosen ist nun zur Verbindung mit der Signal- oder Alarmvorrichtung der Stecker einer entsprechenden Verbindungsleitung einzustecken, wobei es sehr zweckmässig ist, wenn die Signal- oder Alarmanlage so ausgerüstet ist, dass sich mehrere Schaltvorrichtungen parallel anschliessen lassen.
Handelt es sich aber um eine Anlage mit einem dauernd fliessenden Kontroll- oder Sicherheitsstrom, dessen Unterbrechung die Alarm-Auslösung zu bewirken hat, dann sind als Elektroschalter Ausschalter 21 und 22 zu verwenden, die in Serie, also hintereinander zu schalten sind, wie das in der Fig.5 dargestellt ist. In diesem Fall ist zwar in eine der beiden mit 17 bezeichneten Steckdosen der Stecker der zur Verbindung mit der Signal- oder Alarmeinrichtung dienenden Leitung einzustecken, in die andere der beiden Steckdosen 17 ist aber der Kurzschlussstecker 18 einzustecken. Auch bei solchen Anlagen ist es nicht unüblich, dass sie so ausgebildet sind dass mehrere Schalter angeschlossen werden können, bei denen es sich also immer um Ausschalter handelt und die demzufolge alle hintereinander, also in Serie geschaltet sind.

Zur Benützung wird die ganze Schaltvorrichtung so, wie das in der Fig.3 dargestellt ist, an einem Bett angebracht: Zuerst wird der vertikale Schenkel 11 in ein wasserdichtes Futteral 19 gepackt. Dann wird die Platte 1 etwa in der Mitte der Längsseite des Bettes so weit direkt unter die Matratze 20 geschoben, also, falls ein Matratzenschoner vorhanden ist, zwischen Matratze und Matratzenschoner, bis der vertikale Schenkel 11 der Vorrichtung satt an der Seitenfläche der Matratze 20 anliegt und sich nach oben genau so weit erstreckt wie die Matratze. Anschliessend wird die Verbindung mit der Signal- oder Alarmeinrichtung dadurch hergestellt, dass man den Stecker des entsprechenden Verbindungskabels in die Steckdose 16 bzw.17 einsteckt. Zum Schluss wird das Unterleintuch, als welches üblicherweise ein Stretchleintuch 23 verwendet wird, über die Matratze und die Schaltvorrichtung spannt. Nun ist die Schalteinrichtung betriebsbereit: Wenn eine im Bett befindliche Person das Bett verlassen will, wird sie zuerst auf den Bettrand sitzen und durch den Druck auf den vertikal verschiebbaren obern Teil der Schalteinrichtung das gewünschte Signal auslösen.

Wie sich aus der vorstehenden Beschreibung ergibt, weist die vorliegende Erfindung, die sich im privaten Probebetrieb bestens bewährt hat, keine der zahlreichen eingangs beschriebenen, bei den Bettvorlagen vorhandenen Nachteile auf. Zudem ist sie preiswerter herstellbar und besitzt eine gegenüber den Bettvorlagen grössere Lebensdauer.

## Patentansprüche

1. Für ein Bett mit einer Matratze (20) der Dicke h bestimmte Schaltvorrichtung, die anspricht, wenn eine im Bett befindliche Person das Bett verlässt, wobei die Schaltvorrichtung einen vertikalen Schenkel (11) aufweist, der an einer Matratzen-Längsseite anlegbar ist und der vertikale Schenkel (11) aus zwei durch einen horizontalen Zwischenraum voneinander getrennten Teilen (3,10) besteht, von denen der obere (10) entgegen der Kraft mindestens einer Feder (12,13) nach unten verschieb- oder verschwenkbar ist, während im unteren (3) mindestens ein durch die Verschieb- oder Verschwenkbewegung des obern Teiles betätigbarer Elektroschalter (14,15) angeordnet ist, **dadurch gekennzeichnet,**
**dass** die Schaltvorrichtung einen L-förmigen Querschnitt aufweist, dessen vertikaler Schenkel (11) die Länge h und dessen horizontaler Schenkel (1) mindestens die gleiche Länge hat, sodass sie sich zum Gebrauch so am Bett anbringen lässt und der horizontale Schenkel (1) sich unter der Matratze befindet.

2. Schaltvorrichtung nach Anspruch 1, **dadurch gekennzeichnet, dass** im untern Teil (3) je nach Bedarf entweder zwei parallel geschaltete elektrische Einschalter (14,15) oder zwei in Serie geschaltete Ausschalter (21,22) angeordnet sind.

3. Schaltvorrichtung nach einem der Ansprüche 1 oder 2, **dadurch gekennzeichnet, dass** sie an mindestens einem Ende eine 2-polige Steckbuchse (16;17) zum Einstecken eines zum Anschluss an eine Signal- oder Alarm-Anlage dienenden Steckers aufweist.

4. Schaltvorrichtung nach einem der Ansprüche 1 bis 3, **dadurch gekennzeichnet, dass** der obere Teil (10) als Balken mit angenähert quadratischem Querschnitt ausgebildet ist.

5. Schaltvorrichtung nach einem der Ansprüche 1 bis 4, **dadurch gekennzeichnet, dass** sie eine Länge von 50 cm bis 140 cm, vorzugsweise von 80 cm hat.

## Claims

1. A switchgear designated for a bed with a mattress (20) having a thickness h that responds when a person occupying the bed leaves the bed, whereby the switchgear is provided with a vertical bracket (11), that can be attached to the longitudinal side of the mattress and the vertical bracket (11) consists of two parts (3,10) that are separated from each other by a horizontal clearance, of which the upper part (10) can be displaced or can be deviated downward against the force of at least one spring (12,13), while in the lower part (3) an electric switch (14,15) is located that can be actuated by the displacement or deflection motion of the upper part, **characterized by**,
that the switchgear is provided with an L-shaped cross section, the vertical bracket (11) of which has a length h and the horizontal bracket (1) of which has at least the same length, so that it can be attached to the bed for use and the horizontal bracket (1) is under the mattress.

2. Switchgear according to claim 1, **characterized by**, that located in the lower part (3), depending on need, there are either two electric on-switches (14,15) that are connected in parallel, or two off-switches (21,22) that are connected serially.

3. Switchgear according to one of claims 1 or 2, **characterized by**, that it is provided with, at least at one end, a 2-pole socket (16;17) for plugging in a connection to the signal system or alarm system which is served by the plug.

4. Switchgear according to one of claims 1 or 3, **characterized by**, that the upper part (10) is designed as a beam with an almost quadratic cross section.

5. Switchgear according to one of claims 1 to 4, **characterized by**, that it has a length of 50 cm to 140 cm, preferably 80 cm.

## Revendications

1. Dispositif de commutation destiné à un lit possédant un matelas (20) d'épaisseur h et répondant lorsqu'une personne se trouvant dans le lit le quitte, le dispositif de commutation comprenant une branche verticale (11) qui peut reposer sur un grand côté du matelas et la branche verticale (11) étant composée de deux parties (3,10) séparées l'une de l'autre par un espace horizontal, la partie supérieure (10) pouvant être déplacée ou basculée vers le bas contre la force d'au moins un ressort (12,13) tandis que la partie inférieure (3) comporte au moins un commutateur électrique (14,15) qui peut être actionné par le déplacement ou le basculement de la partie supérieure **caractérisé en ce que**
le dispositif de commutation a une section en forme de L dont la branche verticale (11) a une longueur h et dont la branche horizontale (1) a au moins la même longueur, si bien que le dispositif peut ainsi se placer en service sur le lit et que la branche horizontale (1) se trouve au-dessous du matelas.

2. Dispositif de commutation selon la revendication 1, **caractérisé en ce qu'**il est prévu dans la partie inférieure (3), en fonction des besoins, soit deux interrupteurs électriques fermants (14,15) montés en parallèle, soit deux interrupteurs ouvrants (21,22) montés en série.

3. Dispositif de commutation selon l'une des revendications 1 et 2, **caractérisé en ce qu'**il possède à au moins une extrémité une prise de connexion à 2 broches (16;17) pour l'enfichage d'un connecteur destiné au raccordement à une installation de signalement ou d'alarme.

4. Dispositif de commutation selon l'une des revendications 1 à 3, **caractérisé en ce que** la partie supérieure (10) prend la forme d'une poutre de section sensiblement carrée.

5. Dispositif de commutation selon l'une des revendications 1 à 4, **caractérisé en ce qu'**il possède une longueur de 50 cm à 140 cm, de préférence de 80 cm.
